# EUROPEAN PATENT APPLICATION

(11) **EP 3 081 153 A1**
(43) Date of publication of application: **19.10.2016**
(21) Application number: 15164052.1
(22) Date of filing: 17.04.2015
(51) Int. Cl.: A61B 5/00, A61B 5/22, G06F 19/00, A61B 5/0402, A61B 5/11

(54) **BLOOD PRESSURE MEASUREMENT DEVICE ASSOCIATED WITH EVENT**

(71) Applicant: Maisense Inc., Hsinchu County 30273 (TW)
(72) Inventor: Chiu, You-Ming, 302 Zhubei City, Hsinchu County (TW)
(74) Representative: 2K Patentanwälte Blasberg Kewitz & Reichel

(57) **Abstract**

A measurement device (1) for measuring a blood pressure is provided. The measurement device (1) includes at least one sensing sensor, in particular a first sensor (13), a processor (10), and a measurement component (11). The first sensor (13) is used to generate a first sensing signal. The processor (10) receives the first sensing signal and determines whether an event occurs according to the first sensing signal. The measurement component (11) is enabled to measure the blood pressure. When the processor (10) determines that the event occurs, the processor triggers a reminding operation related to enabling of the measurement component (13).

## Description

### BACKGROUND OF THE INVENTION

### FIELD OF THE INVENTION

The invention relates to a measurement device, and more particularly to a measurement device with a reminding operation which can remind a user of measurement of a blood pressure when a specific event occurs.

### DESCRIPTION OF THE RELATED ART

With aging societies, more and more burden is being placed on hospital resources. Moreover, cardiovascular diseases are increasing, as people age and stress increases for modem day living. For example, high blood pressure is a normal symptom of cardiovascular diseases. Thus, blood pressure self-measurement devices become an important target of development in the healthcare industry. Through blood pressure self-measurement manners, patients or users can monitor their own physiology status anytime, to relieve strain on hospital resources and provide needed medical attention to patients.

There have been many blood pressure measurement devices in market, and they are operated independently without any knowledge of relevant events on the users. Therefore, the measurement record cannot help doctors understand the short term change during a day, which can be meaningful to patients. For example, if the patients can measurement their blood pressure after exercise, or after medication, they may compare with their typical blood pressure in the morning, and this is helpful to doctor to make diagnoses or adjust the medication. There is a strong demand to measure and record blood pressure associated with events.

### BRIEF SUMMARY OF THE INVENTION

Thus, it is desirable to provide a measurement device with a reminding operation which can remind a user of measurement of a blood pressure when a specific event occurs, and record the measurement result associated with the event.

An exemplary embodiment of a measurement device for measuring a blood pressure is provided. The measurement device comprises a first sensor, a processor, and a measurement component. The first sensor is used to generate a first sensing signal. The processor receives the first sensing signal and determines whether a pre-defined event occurs according to the first sensing signal. The measurement component is enabled to measure the blood pressure. When the processor determines that the pre-defined event occurs, the processor triggers a reminding operation related to enabling of the measurement component.

An exemplary embodiment of a measurement method for measuring a blood pressure is provided. The measurement method comprises the steps of generating a first sensing signal through a first sensor; determining whether a pre-defined event occurs according to the first sensing signal; and when it is determined that the pre-defined event occurs, triggering a reminding operation related to enabling of measurement of the blood pressure.

Another exemplary embodiment of a measurement device for measuring a blood pressure is provided. The measurement device comprises a measurement component and a processor. The measurement component is enabled to measure the blood pressure. The processor is coupled to the measurement component. The processor enables the measurement component to obtain a measured value of the blood pressure and categorizes the measured value into a specific event.

Another exemplary embodiment of a measurement method for measuring a blood pressure is provided. The measurement method comprises the steps of enabling measurement of the blood pressure to obtain a measured value of the blood pressure and categorizing the measured value into a specific event.

A detailed description is given in the following embodiments with reference to the accompanying drawings.

### BRIEF DESCRIPTION OF THE DRAWINGS

The invention can be more fully understood by reading the subsequent detailed description and examples with references made to the accompanying drawings, wherein:
FIG. 1 shows an exemplary embodiment of a measurement device;
FIG. 2 shows another exemplary embodiment of a measurement device;
FIG. 3 is a flow chart of an exemplary embodiment of a measurement method;
FIG. 4 is a flow chart of another exemplary embodiment of a measurement method; and
FIGs. 5A and 5B are schematic views showing information displayed on a display of an exemplary embodiment of a measurement device.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

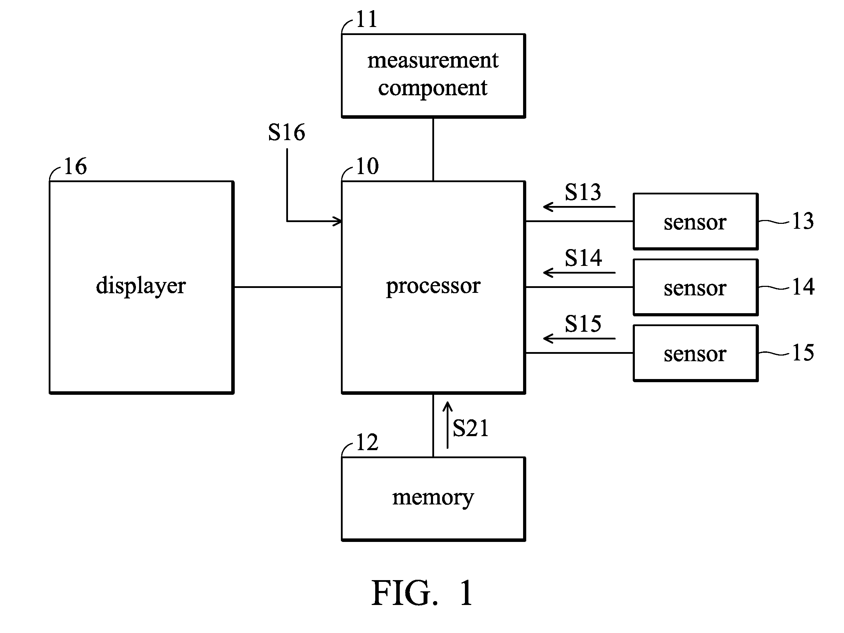

FIG. 1 shows an exemplary embodiment of a measurement device. As shown in FIG. 1, a measurement device 1 is used to measure a blood pressure of a user. The measurement device 1 can be placed on a specific region of an object of the user (such as the wrist or leg) to measure the blood pressure bio-signals through a bracelet. The measurement device 1 comprises a processor 10, a measurement component 11, a memory 12, at least one sensor, and a displayer 16. In the embodiment of FIG. 1, for example, there are three sensors 13-15. The sensors 13-15 are used to sense bio-signals of the user and/or references of the peripheral environments of the user, such as the heart rate of the user or the temperature of the peripheral environments of the user. In this embodiment, for example, the (first) sensor 13 is implemented by a G sensor for sensing the height value of a specific object of the user (such as the chest) and generating a sensing signal S13 according to the sensing result. The (second) sensor 14 is implemented by a heart rate sensor for sensing the heart rate of the user and generating a sensing signal S14 according to the sensing result. The (third) sensor 15 is implemented by a temperature sensor for sensing the temperature of the peripheral environments of the user and generating a sensing signal S15 according to the sensing result. The measurement component 11 can be implemented by a pressure sensor for measuring blood pressure.

The processor 10 receives the sensing signals S13-S15 and determines whether an event occurs according to at least one of the sensing signal S13-S15. When the processor 10 determines that an event occurs, the processor 10 triggers a reminding operation which is performed to remind the user that the blood pressure should be measured. When the user agrees the suggest of the measurement device 1, an indication signal S16 received by the processor 10 is enabled, and the then processor 10 enables the measurement component 11 to measure the blood pressure according to the enabled indication signal S16. That is, the processor 10 enables the measurement of the blood pressure according to the indication signal S16. The detailed operation of the measurement device 1 will be described in the following.

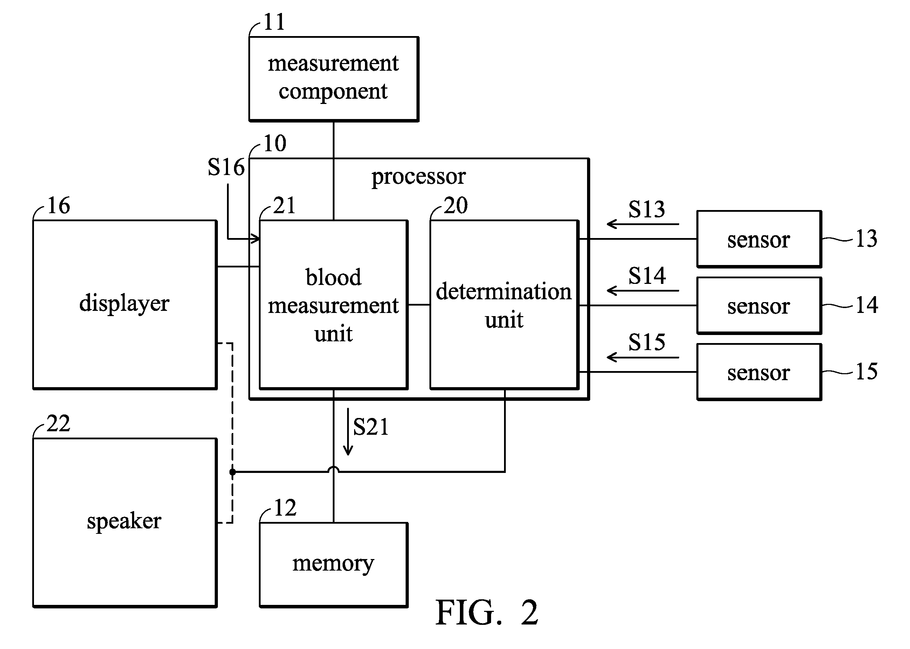

FIG. 2 shows another embodiment of the measurement device 1. As. shown in FIG. 2, the processor 10 comprises a determination unit 20 and a blood measurement unit 21. The determination unit 20 pre-defines a plurality of specific events and different values of the sensing signals S13-S15. One value of each of the sensing signals S13-S15 corresponds to at least one specific event among the pre-defined specific events. The determination unit 20 receives the sensing signals S13-S15 respectively from the G sensor 13, the heart rate sensor 14, and the temperature sensor 15. The determination unit 20 determines whether one of the specific events occurs according to the value of at least one of the sensing signals S13-S15. In an embodiment, when the temperature of the peripheral environments of the user is changed, which is reflected in the value of the signal S 15. Accordingly, the determination unit 20 is able to determine whether an event that the temperature of the peripheral environments of the user is changed occurs according to the sensing signal S15. For example, when the user get into cold environment, the determination unit 20 determines the event that the temperature of the peripheral environments of the user is changed occurs according to the sensing signal S 15. In another embodiment, when the emotion of the user is affected, the heart rate of the user is changed, which is reflected in the value of the signal S14. Accordingly, the determination unit 20 is able to determine whether an event that the emotion of the user is affect occurs according to the sensing signal S14. For example, when the user gets agitated or angry, the determination unit 20 determines the event that the emotion of the user is affected occurs according to the sensing signal S14.

In further another embodiment, when the user takes exercise, the height of the object where the measurement device 1 is placed on the user is continuously changed, which is reflected in the signal S13. Moreover, during the exercise, the heart rate of the user is increased, which is reflected in the value of the signal S14. Accordingly, the determination unit 20 is able to determine whether an event that the user just finishes the exercise occurs according to the sensing signals S 13 and S 14.

When the determination unit 20 determines that a specific event occurs according to the value of at least one of the sensing signals S13-S15, the determination unit 20 records information of the determined specific event in the memory 12. Moreover, when it is determined that the specific event occurs, the determination unit 20 triggers the reminding operation. In the embodiment, when the reminding operation is triggered, the determination unit 20 controls the displayer 16 to show a message related to the enabling of the measurement component 11. For example, the message shown in the displayer 16 includes some words or images which are used to remind or suggest that the blood pressure should be measured. In another embodiment, the measurement device 1 further comprises a speaker 22. When the reminding operation is triggered, the determination unit 20 controls the speaker 22 to play alarm sound related to the enabling of the measurement component 11. For example, the alarm sound played by the speaker 22 is used to remind or suggest that the blood pressure should be measured. In further other embodiments, the reminding operation can be achieved by both of the image shown on the displayer 16 and the alarm sound played by the speaker 22.

After the user is reminded through the message or/and the alarm sound, if the user agrees to measure the blood pressure, the indication signal S16 is enabled by the user, for example, through pressing a bottom disposed on the measurement device 1. The blood pressure unit 21 receives the indication signal S16 and enables the measurement component 11 to measure the blood pressure according to the enabled indication signal S16. When the measurement component 11 finishes the measurement of the blood pressure, the blood pressure unit 21 obtains the value of the blood pressure. The blood pressure unit 21 can control the displayer 16 to show the value of the blood pressure, Moreover, the blood pressure unit 21 can also transmits a blood pressure signal S21 which represents the value of the blood pressure to the memory 12 for record. Accordingly, medical employees can read the memory 12 to be aware of the measured value of the blood pressure and the information of the corresponding specific event which are stored each time when the reminding operation is triggered. In another case, after the user is reminded through the message or/and the alarm sound, if the user does not agree to measure the blood pressure, the indication signal S16 is not enabled by the user, and the blood pressure unit 21 does not enable the measurement component 11 to measure the blood pressure., That is, the measurement of the blood pressure is not enabled.

According to the above embodiments, when a specific event occurs, the measurement device 1 can automatically performs a reminding operation to suggest or remind user to measure the blood pressure. For patients, especially for older patients or patients with cardiovascular diseases, the measurement device 1 with the reminding operation is helpful to blood pressure monitoring and early disease development.

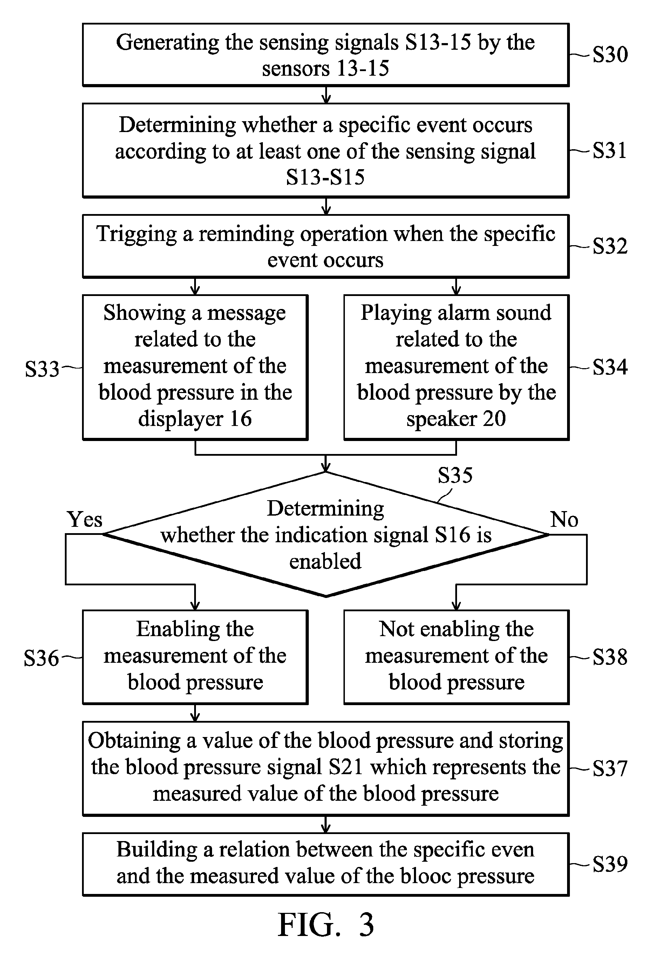

FIG. 3 shows an exemplary embodiment of a measurement method. The measurement method will be described by referring to FIGs. 2 and 3. First, the sensing signals S13-15 are generated by the sensors 13-15 (step S30). It is determined whether a specific event occurs according to at least one of the sensing signal S13-S15 by the determination unit 20 (step S31). In an embodiment, when it is determined that the specific event occurs, a reminding operation is triggered by the determination unit 20 (step S32). In an embodiment, when the reminding operation is triggered, a message related to the measurement of the blood pressure is shown in the displayer 16 (step S33). In another embodiment, when the reminding operation is triggered, alarm sound related to the measurement of the blood pressure is played by the speaker 22 (step S34).

After the message is shown or/and the alarm sound is played, it is determined whether the indication signal S16 is enabled (step S35). If the user agrees to measure the blood pressure, the indication signal S16 is enabled to enable the measurement of the blood pressure (step S36). When the measurement of the blood pressure is enabled, a value of the blood pressure is obtained, and the blood pressure signal S21 which represents the measured value of the blood pressure is stored in the memory 12 (step S37). In another case, after the user is reminded through the message or/and the alarm sound, if the user does not agree to measure the blood pressure, the indication signal S16 is not enabled, and the measurement of the blood pressure is not enabled (step S39). In the step S37, the information of the determined specific event can be recorded in the memory 12. In other embodiments, the information of the determined specific event, such as the name of the determined specific event, can be recorded in the memory 12 just after the determination of the specific event (step S31) is finished.

In the above embodiment, the indication signal S16 is enabled in response to the triggered reminding operation. In other embodiment, the enabling of the indication signal S16 does not relate to the triggered reminding operation. In other words, the indication signal S16 may be enabled when the remaining operation is not triggered In another embodiment, the measurement device 1 may perform an event log operation. The determination unit 20 of the processor 10 pre-sets a plurality of reference events. For example, the reference events comprise occurrences of taking medicine, chest pain, headache, and meal finishing. When the user enables the indication signal S16, the blood pressure unit 21 enables the measurement component 11 to measure the blood pressure. In this embodiment, the indication signal S16 does not relate to the triggered reminding operation. When the measurement component 11 finishes the measurement of the blood pressure, the blood pressure unit 21 obtains the measured value of the blood pressure. The determination unit 20 receives the measured value and categorizes the measured value into a specific event. In the embodiment, the determination unit 20 selects one of the reference evens to a serve as the specific event associated with the measured value. The blood pressure unit 21 can control the displayer 16 to show the measured value of the blood pressure. Moreover, the blood pressure unit 21 can also transmits the blood pressure signal S21 to the memory 12 for record. The determination unit 20 also transmits the information of the specific event to the memory 12 for record. Then, a relation between the specific even and the measured value of the blood pressure is built (step S38). The built relation may be used for monitoring variation in the blood pressure at the specific event.

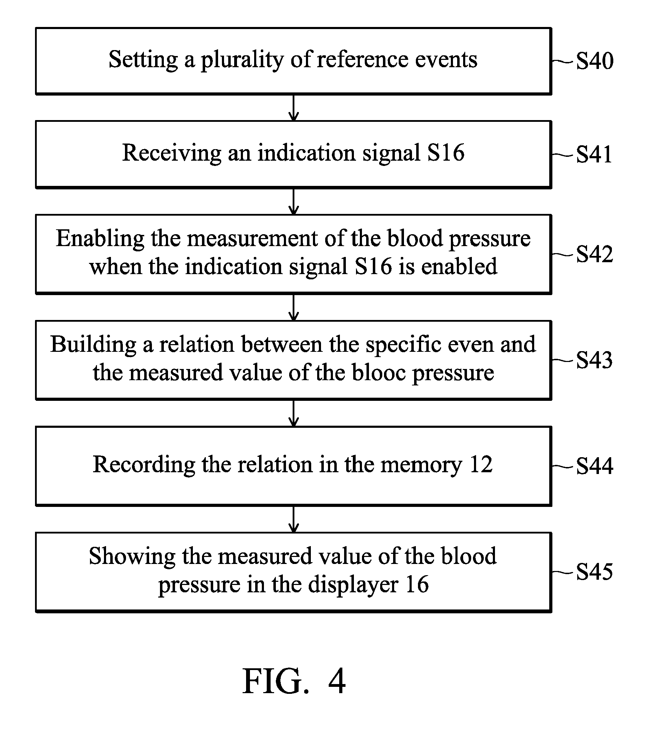

FIG. 4 shows an exemplary embodiment of a measurement method. The measurement method will be described by referring to FIGs. 2 and 4. First, a plurality of reference events are previously set by the determination unit 20 before blood-pressure measurement (step S40). An indication signal S16 is received by the blood pressure unit 21 (step S41). When the indication signal S16 is enabled, the measurement of the blood pressure is enabled by the blood pressure unit 21 (step S42). Then, when the measurement of the blood pressure is finished, the measured value of the blood pressure is obtained, and the measured value of the blood pressure is categorized into one of the reference events by the determination unit 20. In detailed, one of the reference evens is selected by the determination unit 20 according to the measured value of the blood pressure to serve as the specific event associated with the measured value for building a relation between the measured value of the blood pressure and the specific event. The relation is recorded in the memory 12 (step S44) for storage. The relation comprises information about the blood pressure signal S21 which represents the measured value of the blood pressure and about the parameters of the specific event. The built relation may be used for monitoring variation in the blood pressure at the specific event. Moreover, the measured value of the blood pressure can be shown in the displayer 16 (step S45)..

According to the above embodiment, the user can measure the blood pressure and also record the event when the blood pressure is being measured. Through the event log operation, the user or medical employees can easily be aware of the measured value of the blood pressure and the information of the event associated with the measured value. The measurement device 1 with the event log operation is helpful to blood pressure monitoring and early disease development.

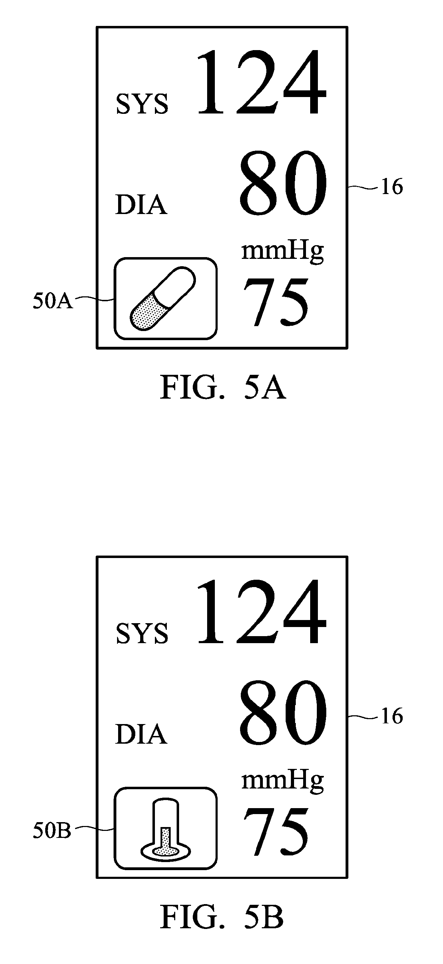

In the above embodiments, the displayer 16 can show the message related to the enabling of the measurement component 11 and the value of the blood pressure. In an embodiment, the displayer 16 can further display the heart rate of the user sensed by the sensor 14. As described above, there is a relation between the specific even and the measured value of the blood pressure. The displayer 16 can also display an image indicating the specific even related to the blood pressure which is displayed on the displayer 16 at the same time. Referring to FIGs. 5A and 5B, the label "SYS" represents the systolic blood pressure with the value "124", the label "DIA" represents the diastolic blood pressure with the value "80", "mmHg" represents the unit of the blood pressure, and the valye "75" represents the heart rate. Moreover, the image "50A" shown in FIG. 5A represents the specific event of occurrences of taking medicine, while the image "50B" shown in FIG. 5B represents the specific even of that the temperature of the peripheral environments of the user is changed. The arrangement of the displayer 16 shown in FIGs. 5A and 5B is an example without limitation for other embodiments.

While the invention has been described by way of example and in terms of the preferred embodiments, it is to be understood that the invention is not limited to the disclosed embodiments. To the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A measurement device (1) for measuring a blood pressure, comprising:
at least one sensor (13-15) generating at least one sensing signal (S13-S15);
a processor (10) receiving the at least one sensing signal and determining whether a pre-defined event occurs according to the at least one sensing signal; and
a measurement component (11) enabled to measure the blood pressure;
wherein when the processor (10) determines that the pre-defined event occurs, the processor triggers a reminding operation related to enabling of the measurement component (11).

2. The measurement device (1) as claimed in claim 1 further comprising:
a displayer (16) coupled to the processor (10);
wherein when the processor triggers the reminding operation, the processer controls the displayer shows a message related to the enabling of the measurement component (11).

3. The measurement device (1) as claimed in claim 1 or 2 further comprising:
a speaker (22) coupled to the processor (10);
wherein when the processor triggers the reminding operation, the processer controls the speaker to play alarm sound related to the enabling of the measurement component (11).

4. The measurement device (1) as claimed in one of the preceding claims further comprising:
a memory (12) coupled to the processor (10);
wherein when the measurement component (11) is enabled to measure the blood pressure according to the reminding operation, the processor stores a measured value of the blood pressure into the memory.

5. The measurement device (1) as claimed in one of the preceding claims, wherein after the processor (10) triggers the reminding operation, the processor enables the measurement component (11) to measure the blood pressure when the processor receives an indication signal.

6. The measurement device (1) as claimed in one of the preceding claims,
wherein the processor (10) receives the at least one sensining signal, in particular a first sensing signal, and determines whether the pre-defined event occurs according to the at least one or first sensing signal, and when the processor (10) determines that the pre-defined event occurs, the processor triggers the reminding operation; and
wherein when the processor triggers the reminding operation and receives an indication signal, the processor (10) enables the measurement component (11) to measure the blood pressure.

7. The measurement device as claimed one of the preceding in claims, wherein when the processor (10) determines that the pre-defined event does not occur and the processor receives an indication signal, the processor enables the measurement component to measure the blood pressure according to the indication signal.

8. The measurement device (1) as claimed in one of the preceding claims, wherein the at least one sensor comprises a G-sensor, a heart rate sensor, and/or or a temperature sensor.

9. The measurement device (1) as claimed in one of the preceding claims, wherein the processor (10) enables the measurement component (11) to obtain a measured value of the blood pressure and categorizes the measured value into a specific event.

10. The measurement device (1) as claimed in one of the preceding claims, wherein wherein when the processor (10) obtains the measured value, the processor controls a or the displayer (16) shows the measured value.

11. The measurement device (1) as claimed in one of the preceding claims 1,
wherein the processor (10) sets a plurality of reference events, in particular reference events comprising occurrences of taking medicine, chest pain, headache, and/or finishing meal; and
when the processor obtains the measured value, the processor selects one of the reference events to serve as the specific event, in particular.

12. A measurement device for measuring a blood pressure, comprising:
a measurement component enabled to measure the blood pressure; and
a processor coupled to the measurement component;
wherein the processor enables the measurement component to obtain a measured value of the blood pressure and categorizes the measured value into a specific event.

13. The measurement device as claimed in claim 12 further comprising:
a displayer coupled to the processor;
wherein when the processor obtains the measured value, the processor controls the displayer shows the measured value.

14. The measurement device as claimed in claim 12 or 13, wherein the processor sets a plurality of reference events, and when the processor obtains the measured value, the processor selects one of the reference events to serve as the specific event.

15. The measurement device as claimed in claim 14, wherein the reference events comprise occurrences of taking medicine, chest pain, headache, and finishing meal.

## Amended claims

### Amended claims in accordance with Rule 137(2) EPC.

1. A measurement device (1) for measuring a blood pressure, comprising:
a measurement component (11) to measure the blood pressure; and
a processor (10) coupled to the measurement component;
wherein the processor enables the measurement component to obtain a measured value of the blood pressure and categorizes the measured value into a specific event.

2. The measurement device (1) as claimed in claim 1 further comprising:
a displayer (16) coupled to the processor (10);
wherein when the processor (10) obtains the measured value of the blood pressure, the processor (10) controls the displayer (16) to show the measured value of the blood pressure and the specific event.

3. The measurement device as claimed in claim 1 or 2, wherein the processor (10) sets a plurality of reference events, and when the processor (10) obtains the measured value of the blood presure, the processor (10) selects one of the reference events to serve as the specific event.

4. The measurement device as claimed in claim 3, wherein the reference events comprise occurrences of taking medicine, chest pain, headache, or finishing meal.

5. The measurement device (1) as claimed in claim 1 further comprising:
at least one sensor (13-15) generating at least one sensing signal (S13-S15);
wherein the processor (10) receives the at least one sensing signal and
determines whether a pre-defined event occurs according to the at least one sensing signal;
wherein when the processor (10) determines that the pre-defined event occurs according to the at least one sensing signal and receives an indication signal input from an input element, the processor enables the measurement component (11) to measure the blood pressure to obtain the measured value.

6. The measurement device (1) as claimed in claim 5 further comprising:
a displayer (16) coupled to the processor (10);
wherein when the processor determines that the pre-defined event occurs, the processer controls the displayer shows a message informing a user to measure the blood pressure.

7. The measurement device (1) as claimed in claim 5 further comprising:
a speaker (22) coupled to the processor (10);
wherein when the processor determines that the pre-defined event occurs, the processer controls the speaker to play alarm sound informing a user to measure the blood pressure.

8. The measurement device as claimed in claim 5, wherein when the processor (10) determines that the pre-defined event does not occur and the processor receives the indication signal, the processor enables the measurement component to measure the blood pressure according to the indication signal.

9. The measurement device (1) as claimed in claim 5, wherein the at least one sensor comprises a G-sensor, a heart rate sensor, or a temperature sensor.

10. The measurement device (1) as claimed in claim 1 further comprising:
a memory (12) coupled to the processor (10);
wherein the processor stores the measured value and specific event of the blood pressure into the memory.
